# EUROPEAN PATENT SPECIFICATION

(11) **EP 3 263 084 B1**
(45) Date of publication and mention of the grant of the patent: **25.03.2020**
(21) Application number: 16207369.6
(22) Date of filing: 29.12.2016
(51) Int. Cl.: A61G 7/057, A61B 5/00, A61F 7/00

(54) **MICROCLIMATE MANAGEMENT SYSTEM WITH WIRELESS SENSORS**
MIKROKLIMAVERWALTUNGSSYSTEM MIT DRAHTLOSEN SENSOREN
SYSTÈME DE GESTION DE MICROCLIMAT AYANT DES CAPTEURS SANS FIL

(30) Priority: 01.07.2016 US 201662357554 P
(43) Date of publication of application: 03.01.2018
(73) Proprietor: Hill-Rom Services, Inc., Batesville, IN 47006-9167 (US)
(72) Inventor: COLEMAN II, Leigh Scott, Batesville, Indiana 47006-9167 (US); KAIKENGER, Philippe, Batesville, Indiana 47006-9167 (US); KING, Catherine, Batesville, Indiana 47006-9167 (US); LAI, Samuel, Batesville, Indiana 47006-9167 (US); SALIBRA, Alisa Robinson, Batesville, Indiana 47006-9167 (US); STEBBINS, Kristen L., Batesville, Indiana 47006-9167 (US); TALLENT, Dan R, Batesville, Indiana 47006-9167 (US); VENTROLA, Todd, Batesville, Indiana 47006-9167 (US); WILLIAMSON, Rachel, Batesville, Indiana 46007-9167 (US)
(74) Representative: Findlay, Alice Rosemary

(56) References cited:
- WO-A1-2009/129306
- US-A1- 2005 267 339
- US-A1- 2009 149 927
- US-A1- 2011 263 950
- Charlie Lachenbruch: "Microclimate Management So much more than just airflow...", , 1 January 2008 (2008-01-01), XP055550311, Retrieved from the Internet: URL:https://cmapspublic3.ihmc.us/rid=1J2LQ 3LQT-1ZKHCLT-V0D/10-Microclimate%20Managem ent.pdf [retrieved on 2019-01-31]

## Description

Patients lying on support devices, such as hospital bed mattresses, for extended periods of time are susceptible to the development of pressure ulcers (also known as decubitus ulcers or bedsores). Pressure ulcers are lesions often found adjacent bony or cartilaginous areas. Pressure ulcers may be caused by tissue forces, such as, for example, pressure, i.e., compression of tissues, shear force, and friction. Pressure ulcer formation may be exacerbated by the presence of excess body heat and/or moisture.

US 2011/0263950 discloses a system for monitoring medical conditions including pressure ulcers, pressure-induced ischemia and related medical conditions comprises at least one sensor adapted to detect one or more patient characteristic including at least position, orientation, temperature, acceleration, moisture, resistance, stress, heart rate, respiration rate, and blood oxygenation.

WO2009/129306 discloses a microclimate management system with a topper on top of a mattress and a control system. The control system is configured to maintain a surface temperature, or relative surface humidity, or a heat withdrawal capacity of the surface.

The invention is defined by the claims.

The invention will now be further described by way of example with reference to the accompanying drawings, in which:
Figure 1 shows an example environment including a patient support device and a microclimate management system.
Figure 2 shows additional details of the environment of Figure 1.
Figure 3 shows an example process diagram illustrating possible operation within the environment of Figure 1.
Figure 4 shows another example process diagram illustrating possible operation within the environment of Figure 1.
Figure 5 shows example physical components of the microclimate management system of Figure 1.

The present disclosure is directed to systems and methods for manipulating the environment for a patient in an ambulatory environment, such as a hospital or clinic setting. In some examples, the temperature of the patient is measured using wireless sensors at one or more points on the patient. A microclimate management system receives those temperature readings and can modify the environment surrounding the patient, such as temperature, relative humidity, etc.

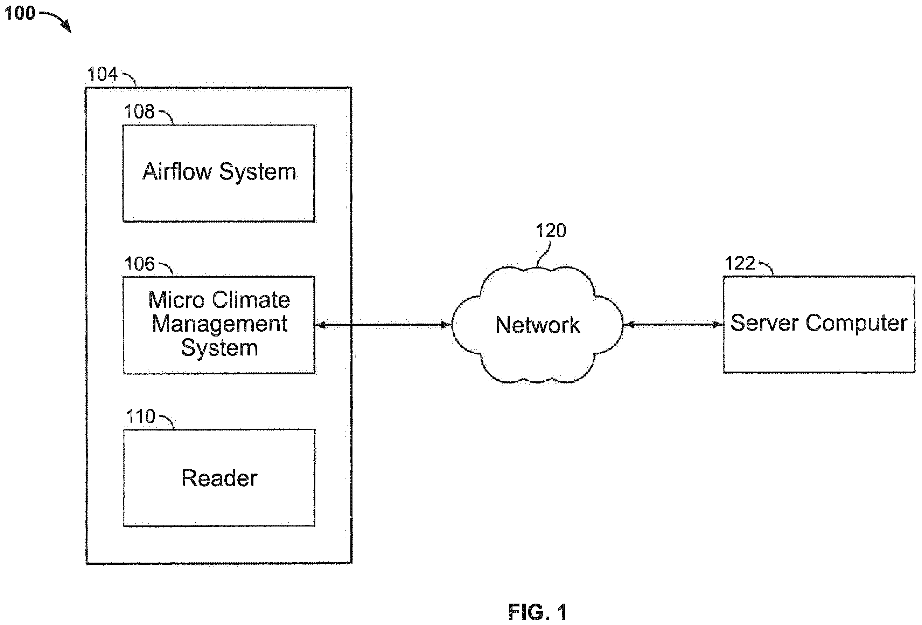

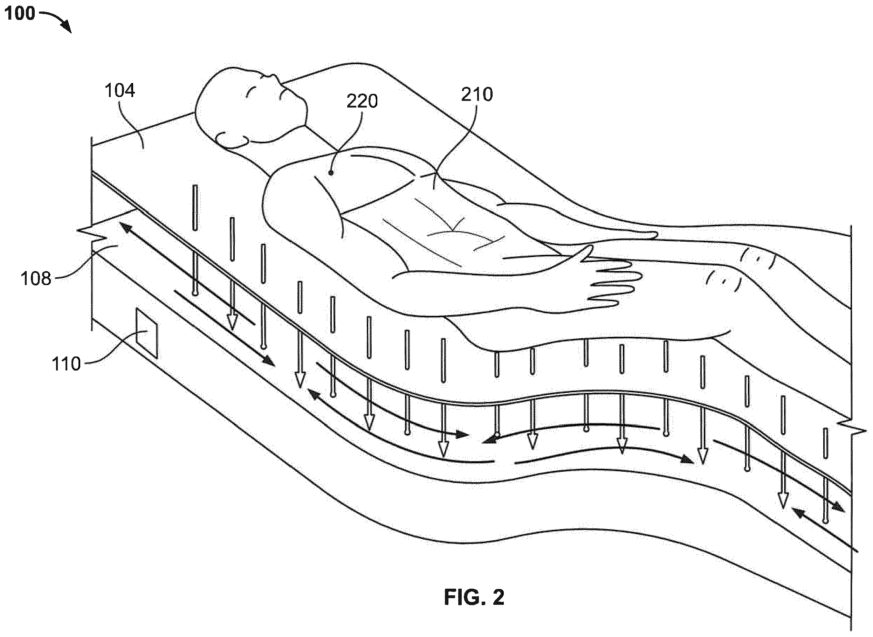

Figures 1-2 show an example environment 100, such as a hospital or clinical setting. In this context, a patient support device 104, such as a mattress, provides support for a patient 210. One example of such a patient support device 104 is the TotalCare® P500 Intensive Care Bed manufactured by Hill-Rom of Batesville, Indiana. Other configurations are possible.

The patient support device 104 includes a microclimate management system 106 associated with the patient support device 104. The microclimate management system 106 is configured to monitor and modify the environment surrounding the patient 210, as described further herein. For example, the microclimate management system 106 can be configured to modify the temperature and/or relative humidity surrounding the patient 210 to achieve desired clinical benefits.

To accomplish this functionality, the microclimate management system 106 controls an airflow system 108 associated with the patient support device 104. In this example, the microclimate management system 106 can modify an amount of air flowing through the airflow system 108 and/or a temperature and humidity of the air flowing therethrough. This, in turn, can modify a temperature of the patient 210 that is supported by the patient support device 104.

In one example, the microclimate management system 106 is configured in a manner described in U.S. Patent Published Application No. 2011/0024076 A1 to Lachenbruch et al. The Application discloses detailed examples of microclimate management systems.

The patient support device 104 is also associated with a reader 110 positioned within or adjacent to the patient support device 104. In this example, the reader 110 is configured to read information from one or more sensors associated with the patient 210.

For example, in one embodiment, one or more wireless patches or wireless sensors 220 are placed at desired locations on the body of the patient 210. For example, each wireless sensor 220 can includes an adhesive portion that removable couples the wireless sensor 220 to the skin of the patient 210. The wireless sensor 220 can also include various components, such as a thermistor, a processor, memory, and/or a source of power like a battery. In other examples, the wireless sensor 220 can be without its own source of power (i.e., passive).

Although a single wireless sensor 220 is shown, multiple sensors can be used and positioned at different areas of the body of the patient 210. For example, multiple temperature sensors can be positioned at different parts of the body of the patient 210 to provide a more localized temperature. If, for instance, the hands and feet of the patient 210 are cold, then the heating could be increased (and vice versa if the hands and feet are warm). Likewise, other sensors, such as a galvanic skin response sensor that measures the electrical conductance of the skin, could be used to sense sweating by the patient 210, which would be an indication that the environment is too warm.

Further, multiple readers can be provided along the patient support device 104, if desired.

In this example, the wireless sensor 220 is configured to measure a temperature of the patient 210 at the positioned location. The temperature can be stored, and the reader 110 is configured to interrogate the wireless sensor 220 at desired intervals to read the measured temperature and communicate that temperature to the microclimate management system 106.

For example, the reader 110 can use different communication schemes, such as nearfield communication (NFC) to interrogate the wireless sensor 220. The reader 110 can be positioned within or adjacent to the patient support device 104 such that the reader 110 is in close enough proximity to read the wireless sensor 220.

In example embodiments, the wireless sensor 220 is configured in the manner described in U.S. Patent Application Serial No. 15/053,661 to Quinn. Other configurations are possible.

The reader 110 is programmed to communicate the temperature reading(s) to the microclimate management system 106. The microclimate management system 106, in turn, uses the temperature information to maintain the patient 210 within a desired environment. For example, if the temperature reading is high, the microclimate management system 106 can modify the temperature, humidity, and/or speed of the air flowing in the airflow system 108 in order to reduce a temperature of the patient 210.

In another example, the microclimate management system 106 can be programmed to manage temperature readings from multiple locations on the body of the patient 210 and modify airflow to areas associated various portions of the body to maintain desired temperatures at specific locations on the body. Further, the microclimate management system 106 can monitor a trend of the temperature(s) over time and use feedback mechanisms to accomplish a desired temperature, as described further below.

The microclimate management system 106 can also communicate the temperature information to a remote system, such as a server computer 122, through a network 120. For example, the server computer 122 can be a central server, such as caregiver station, that allows a caregiver to monitor the temperature reading(s) for the patient 210. Further, various alerting can be provided to the caregiver, such as if the temperature falls outside a given range. In another example, the server computer 122 can be an electronic medical record (EMR) repository, and the temperature reading(s) can be captured within the EMR for the patient 210. Other configurations are possible.

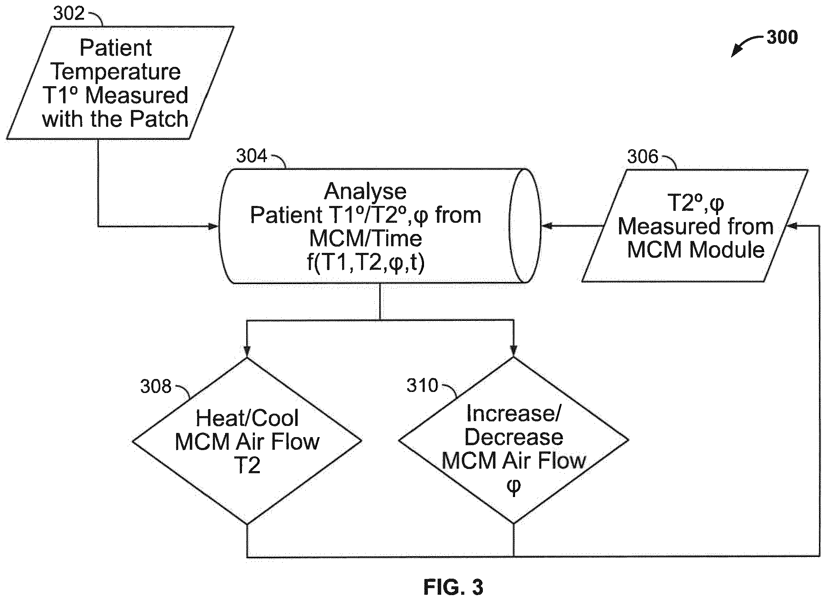

Referring now to Figure 3, an example process diagram 300 illustrating the operation within the environment 100 is shown.

At process 302, the patient's temperature (T1) is measured by the one or more wireless sensor 220. This is accomplished, as described above, by the wireless sensors taking one or more temperature measurements on the patient's body.

At process 306, the temperature (T2) and/or relative humidity of the air and the speed of the airflow (ϕ) being provided by the microclimate management system is determined.

At process 304, the microclimate management system analyzes this information over time (t): f(T1, T2, ϕ, t).

At process 308, the microclimate management system can heat or cool the air flowing through the airflow system to increase or decrease the temperature of the patient. This can be done based upon an average of the temperatures provided by the wireless sensors (if more than one is used) and/or different zones within the airflow system can be used to increase or decrease the temperature at specific areas adjacent to the patient's body.

Likewise, at process 310, the microclimate management system can modify the speed of the airflow through the airflow system to accomplish desired heating or cooling.

The temperatures T1, T2 and airflow speed ϕ can be monitored over time, and the microclimate management system can modify heating or cooling (or airflow speed) as desired based upon these measurements. This modification can be automated. In other examples, the modifications can be suggested to the caregiver, such as on a graphical user interface. The caregiver can then manually adjust the microclimate management system based upon the suggestions.

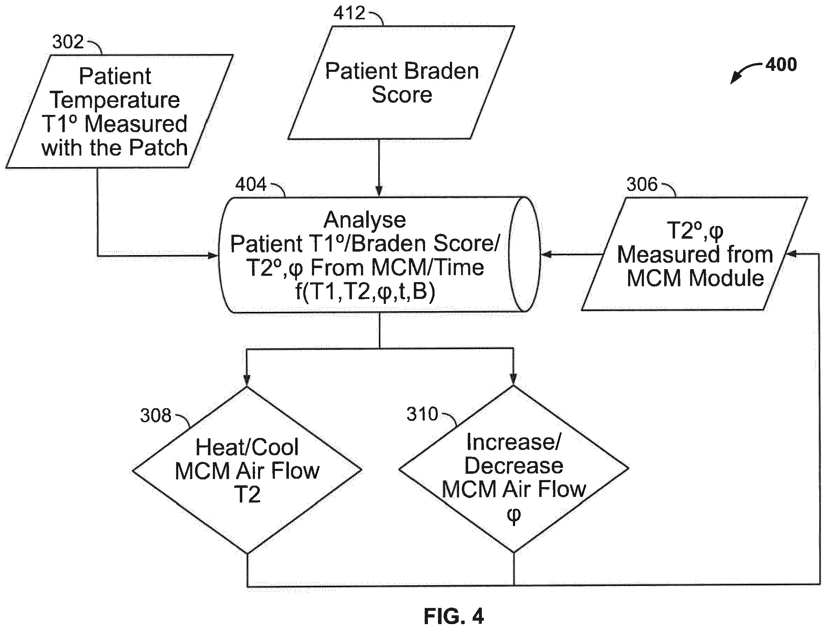

Referring now to Figure 4, another example process diagram 400 illustrating the operation within the environment 100 is shown.

The process diagram 400 is similar to the process diagram 300 described above, except that the patient's Braden score is determined at process 412. The Braden score is derived form a Braden Scale for Predicting Pressure Ulcer Risk, which is a tool that is used to measure the patient's risk of developing a pressure ulcer. Factors such as moisture of the skin, activity, mobility, nutrition, and friction/shear can be factored into deriving the score.

In one example, the Braden score is manually determined and provided to the microclimate management system. In another example, one or more automated processes (e.g., sensors used to collect factors like moisture, activity, etc.) are used to derive the Braden score.

At operation 404, the microclimate management system analyzes the Braden score (B) as part of the information used to determine modifications to the airflow temperature and speed: f(T1, T2, ϕ, t, B). The Braden score can be used as a check for the environment 100. For example, too much warming can be associated with pressure ulcer events - using the Braden score as one input into the system can help to assure that excessive heating is not provided by the microclimate management system.

In the examples provided herein, the various components of the environment 100 can be implemented as one or more computing devices. For example, the microclimate management system 106 can be a computing device. Other components, such as the reader, server computer, and wireless sensors, can also be implemented as one or more computing devices.

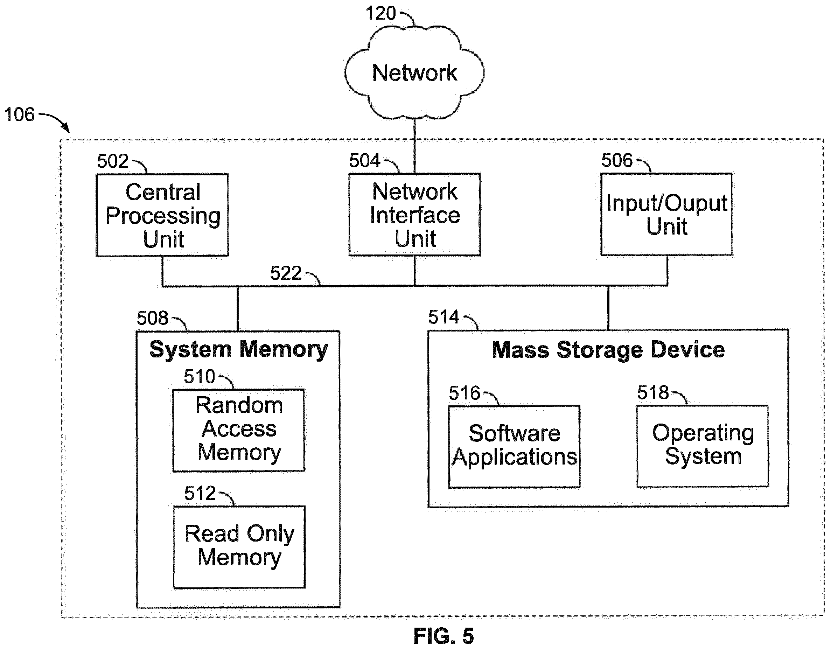

As illustrated in Figure 5, the microclimate management system 106 includes at least one central processing unit ("CPU") 502, a system memory 508, and a system bus 522 that couples the system memory 508 to the CPU 502. The system memory 508 includes a random access memory ("RAM") 510 and a read-only memory ("ROM") 512. A basic input/output system contains the basic routines that help to transfer information between elements within the microclimate management system 106, such as during startup, is stored in the ROM 512. The microclimate management system 106 further includes a mass storage device 514. The mass storage device 514 is able to store software instructions and data. A central processing unit, system memory and mass storage device similar to that in Figure 5 are also included in server computer 102.

The mass storage device 514 is connected to the CPU 502 through a mass storage controller (not shown) connected to the system bus 522. The mass storage device 514 and its associated computer-readable data storage media provide non-volatile, non-transitory storage for the microclimate management system 106. Although the description of computer-readable data storage media contained herein refers to a mass storage device, such as a hard disk or solid state disk, it should be appreciated by those skilled in the art that computer-readable data storage media can be any available non-transitory, physical device or article of manufacture from which the central display station can read data and/or instructions.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the microclimate management system 106.

According to various embodiments, the microclimate management system 106 may operate in a networked environment using logical connections to remote network devices through the network 120, such as a wireless network, the Internet, or another type of network. The microclimate management system 106 may connect to the network 120 through a network interface unit 504 connected to the system bus 522. It should be appreciated that the network interface unit 504 may also be utilized to connect to other types of networks and remote computing systems. The microclimate management system 106 also includes an input/output controller 506 for receiving and processing input from a number of other devices, including a touch user interface display screen, or another type of input device. Similarly, the input/output controller 506 may provide output to a touch user interface display screen or other type of output device.

As mentioned briefly above, the mass storage device 514 and the RAM 510 of the microclimate management system 106 can store software instructions and data. The software instructions include an operating system 518 suitable for controlling the operation of the microclimate management system 106. The mass storage device 514 and/or the RAM 510 also store software instructions, that when executed by the CPU 502, cause the microclimate management system 106 to provide the functionality of the microclimate management system 106 discussed in this document. For example, the mass storage device 514 and/or the RAM 510 can store software instructions that, when executed by the CPU 502, cause the microclimate management system 106 to adjust a temperature and/or speed of airflow being provided to the patient.

Although various embodiments are described herein, those of ordinary skill in the art will understand that many modifications may be made thereto.

## Claims

1. A system for monitoring an environment for a patient on a patient support device, the system comprising:
a patient support device (104),
multiple wireless sensors (220) configured to be placed on a body of the patient, including multiple temperature sensors, and a galvanic skin response sensor to detect sweating on the body of the patient;
a controller programmed to receive temperature readings from the multiple wireless temperature sensors (220) and the galvanic skin response sensor; and
an airflow system (108), associated with the patient support device (104), and configured to provide airflow within the patient support device (104), wherein the controller is programmed to:
determine the temperature of the air and the speed of the airflow being provided by the airflow system;
analyse the temperature readings, the galvanic skin response reading, the temperature of the air, and the airflow speed, over time; and
modify air flowing through the airflow system (108), based on the analysis, to maintain a patient within a desired environment.

2. The system of claim 1, wherein the multiple wireless sensors (220) are positionable at different areas of the body.

3. The system of claim 2, wherein the controller is programmed to modify the air flowing through the airflow system (108) based upon the temperature readings at the different areas of the body.

4. The system of any preceding claim, wherein the controller is programmed to increase or decrease an air temperature or an air speed of the air flowing through the airflow system (108) when sweating is detected.

5. The system of any preceding claim, wherein the controller is programmed to adjust an air speed of the air flowing through the airflow system (108).

6. The system of any preceding claim, wherein the controller is programmed to use a Braden score when comparing the temperature to the air flowing through the airflow system (108).

## Patentansprüche

1. Ein System zur Überwachung einer Umgebung für einen Patienten auf einer Patiententragevorrichtung, wobei das System umfasst:
eine Patiententragevorrichtung (104),
mehrere drahtlose Sensoren (220), die zum Anlegen am Körper eines Patienten konfiguriert sind, einschließlich mehrerer Temperatursensoren, und einen Sensor für eine galvanische Hautreaktion, um Schwitzen am Körper des Patienten festzustellen;
eine Steuereinheit, die zum Empfang der Temperaturmesswerte von den mehreren drahtlosen Temperatursensoren (220) und dem Sensor für eine galvanische Hautreaktion programmiert ist; und
ein Luftstromsystem (108), das zu der Patiententragevorrichtung (104) gehört, und so konfiguriert ist, dass ein Luftstrom innerhalb der Patiententragevorrichtung (104) bereitgestellt wird, wobei die Steuereinheit so programmiert ist, dass:
die Temperatur der Luft und die Geschwindigkeit des von dem Luftstromsystem bereitgestellten Luftstroms ermittelt wird;
die Temperaturmesswerte, der Messwert für die galvanische Hautreaktion, die Temperatur der Luft und die Luftstromgeschwindigkeit im Laufe der Zeit analysiert werden; und
die durch das Luftstromsystem (108) strömende Luft auf Grundlage der Analyse verändert wird, um einen Patienten innerhalb einer gewünschten Umgebung zu behalten.

2. Das System nach Anspruch 1, wobei die mehreren drahtlosen Sensoren (220) an verschiedenen Bereichen des Körpers positionierbar sind.

3. Das System nach Anspruch 2, wobei die Steuereinheit so programmiert ist, dass die durch das Luftstromsystem (108) strömende Luft auf Grundlage der Temperaturmesswerte in den verschiedenen Bereichen des Körpers verändert wird.

4. Das System nach einem der vorstehenden Ansprüche, wobei die Steuereinheit so programmiert ist, dass eine Lufttemperatur oder eine Luftgeschwindigkeit der durch das Luftstromsystem (108) strömenden Luft erhöht oder vermindert wird, wenn Schwitzen festgestellt wird.

5. Das System nach einem der vorstehenden Ansprüche, wobei die Steuereinheit so programmiert ist, dass eine Luftgeschwindigkeit der durch das Luftstromsystem (108) strömenden Luft angepasst wird.

6. Das System nach einem der vorstehenden Ansprüche, wobei die Steuereinheit so programmiert ist, dass ein Braden-Punktwert beim Vergleich der Temperatur mit der durch das Luftstromsystem (108) strömenden Luft verwendet wird.

## Revendications

1. Système de contrôle d'un environnement pour un patient sur un dispositif de support de patient, le système comprenant :
un dispositif de support de patient (104),
de multiples capteurs sans fil (220) configurés pour être placés sur un corps du patient, incluant de multiples capteurs de température, et un capteur de réflexe cutané galvanique pour détecter la transpiration sur le corps du patient ;
un dispositif de commande programmé pour recevoir les relevés de température des multiples capteurs de température sans fil (220) et du capteur de réflexe cutané galvanique ; et
un système d'écoulement d'air (108), associé au dispositif de support de patient (104), et configuré pour fournir un écoulement d'air à l'intérieur du dispositif de support de patient (104), dans lequel le dispositif de commande est programmé pour :
déterminer la température de l'air et la vitesse de l'écoulement d'air fourni par le système d'écoulement d'air ;
analyser les relevés de température, le relevé du réflexe cutané galvanique, la température de l'air, et la vitesse d'écoulement d'air, dans le temps ; et
modifier l'écoulement d'air à travers le système d'écoulement d'air (108), en fonction de l'analyse, pour maintenir un patient dans un environnement désiré.

2. Système selon la revendication 1, dans lequel les multiples capteurs sans fil (220) sont positionnables sur différentes parties du corps.

3. Système selon la revendication 2, dans lequel le dispositif de commande est programmé pour modifier l'écoulement d'air qui passe dans le système d'écoulement d'air (108) en fonction des relevés de température sur les différentes zones du corps.

4. Système selon l'une quelconque des revendications précédentes, dans lequel le dispositif de commande est programmé pour augmenter ou diminuer la température de l'air ou une vitesse de l'air qui passe dans le système d'écoulement d'air (108) lorsque de la transpiration est détectée.

5. Système selon l'une quelconque des revendications précédentes, dans lequel le dispositif de commande est programmé pour ajuster une vitesse d'air de l'air qui passe dans le système d'écoulement d'air (108).

6. Système selon l'une quelconque des revendications précédentes, dans lequel le dispositif de commande est programmé pour utiliser un score Braden lors de la comparaison entre la température et l'air qui passe dans le système d'écoulement d'air (108).
